# EUROPEAN PATENT SPECIFICATION

(11) **EP 2 374 834 B1**
(45) Date of publication and mention of the grant of the patent: **01.08.2018**
(21) Application number: 09830462.9
(22) Date of filing: 27.11.2009
(51) Int. Cl.: C08J 3/12, A61K 8/88, A61Q 1/00, B29C 67/00, B29B 7/88, B29B 7/90, A61K 8/27, A61K 8/29, A61Q 1/02, A61Q 17/04, A61K 8/02

(54) **SPHERICAL COMPOSITE PARTICLES AND MANUFACTURING METHOD for manufacturing the same**
KUGELFÖRMIGE VERBUNDPARTIKEL UND HERSTELLUNGSVERFAHREN DAFÜR
PARTICULES COMPOSITES SPHÉRIQUES ET PROCÉDÉ DE FABRICATION ASSOCIÉ

(30) Priority: 05.12.2008 JP 2008311266
(43) Date of publication of application: 12.10.2011
(73) Proprietor: Daicel-Evonik Ltd., Shinjuku-ku, Tokyo 163-0912 (JP)
(72) Inventor: MATSUI, Hideki, Himeji-shi Hyogo 671-1281 (JP); NAKAIE, Yoshiki, Himeji-shi Hyogo 671-1281 (JP); KOMADA, Hajime, Himeji-shi Hyogo 671-1281 (JP)
(74) Representative: Grünecker Patent- und Rechtsanwälte PartG mbB
(86) International application number: PCT/JP2009/070366
(87) International publication number: WO 2010/064696

(56) References cited:
- EP-A1- 1 982 816
- WO-A1-2005/052034
- WO-A1-2008/149755
- JP-A- 60 040 134
- JP-A- 61 009 433
- JP-A- H10 504 045
- JP-A- 2001 114 901
- JP-A- 2001 199 836
- JP-A- 2005 200 663
- JP-A- 2006 126 359
- JP-A- 2006 321 711
- JP-A- 2007 210 335
- JP-A- 2007 277 546
- FEDORS ROBERT F: "A METHOD FOR ESTIMATING BOTH THE SOLUBILITY PARAMETERS AND MOLAR VOLUMES OF LIQUIDS", POLYMER ENGINEERING AND SCIENCE, BROOKFIELD CENTER, US, vol. 14, no. 2, 1 February 1974 (1974-02-01), pages 147-154, XP008069569, ISSN: 0032-3888, DOI: 10.1002/PEN.760140211

## Description

### Technical Field

The present invention relates to a method for manufacturing spherical composite particles composed of an organic solid containing a thermoplastic or thermosetting resin; and the manufactured spherical composite particles. More specifically, the present invention relates to a method for manufacturing spherical composite particles and the resulting spherical composite particles, which spherical composite particles are used in powder coatings, cosmetics, and toners or used in forming techniques such as slush molding and powder-layered manufacturing.

### Background Art

An exemplary known method for manufacturing resin particles is a method for manufacturing particles of a thermoplastic resin, which involves heating and melting the thermoplastic resin and another resin immiscible with the thermoplastic resin; and washing the resulting article with a solvent (Patent Literature (PTL) 1). An exemplary known method for manufacturing a cosmetic containing an inorganic filler and a colorant is a method for manufacturing a cosmetic, which involves melting and kneading a resin for constituting a powder, a medium for dispersing the resin, and an inorganic pigment to form resin particles composed of a powder internally including the inorganic pigment (PTL 2).

In general, including-type resin particles which internally include inorganic microparticles may be easily manufactured by preliminarily compounding an inorganic pigment into a resin to be powdered, and melting and kneading the compound with another resin working as a medium. This method, however, requires two or more kneading steps and thereby suffers from complicated operations, leading to increased cost. In contrast, outer-bearing-type resin particles, which are composed of resin particles and inorganic microparticles immobilized to the outer surface of the resin particles, are capable of exhibiting high functions of the inorganic microparticles without deterioration. However, such customary manufacturing methods as mentioned above may fail to manufacture target resin particles externally bearing inorganic microparticles, because considerable amounts of the inorganic microparticles are incorporated in the inside of the resin particles. Naturally these methods fail to control the incorporation of the inorganic microparticles to outside and inside of the resin particles.

JP 2007210335 A and JP 2007-277546 A disclose methods for manufacturing spherical composite particles.

### Citation List

### Patent Literature

PTL 1: Japanese Unexamined Patent Application Publication (JP-A) No. S61(1986)-9433
PTL 2: Japanese Unexamined Patent Application Publication (JP-A) No. 2001-199836

### Summary of Invention

### Technical Problem

An object of the present invention is to provide a method for manufacturing spherical composite particles, by which desired function(s) can be simply and efficiently imparted to the surface and/or inside of the spherical composite particles. Another object of the present invention is to provide spherical composite particles obtained by the method.

### Solution to Problem

After intensive investigations to achieve the objects, the present inventors have found that the use of a filler undergone a specific surface treatment stably and easily gives an organic solid powder (organic fine solid particles) internally including or externally bearing the filler; and the use of such fillers of different types stably and easily gives an organic solid powder internally including a filler and externally bearing another filler. The present invention has been made based on these findings.

Specifically, the present invention provides, in one aspect, a method for manufacturing spherical composite particles, the method comprising:
melting and kneading a meltable organic solid (A), a component (B) immiscible with the organic solid (A), and one or more fillers (C) having specific differences in solubility parameter SP from the organic solid (A) to give spherical composite particles comprising the organic solid (A) and the filler(s) (C) and being dispersed in the component (B),
wherein the meltable organic solid (A) is selected from thermoplastic resins, unvulcanized rubbers and thermoplastic elastomers,
wherein a first filler having a difference in SP of less than 5 and a second filler having a difference in SP of 5 or more are used as the fillers (C) to give the spherical composite particles which internally include the first filler (C) and externally bear the second filler (C),
wherein the filler(s) (C) comprises a filler whose surface has been treated with a substance having a specific difference in SP from the organic solid (A), and
wherein the solubility parameter SP is determined according to the technique described in "SP values, basis, applications, and calculating methods; Hideki Yamamoto, Johokiko Co. Ltd., issued on March 31, 2005".

The filler(s) (C) preferably includes, at least at its surface, a filler having a specific difference in SP from the organic solid (A). Alternatively, the filler(s) (C) preferably includes a filler whose surface has been treated with a substance having a specific difference in SP from the organic solid (A). The organic solid (A) is preferably a thermoplastic resin; and the thermoplastic resin is preferably a polyamide resin. The filler (s) (C) preferably has at least one hydrophilic group such as carboxy group, carboxylic acid group, a carboxylic acid salt, amino group, amide group, or epoxy group. Alternatively, the filler(s) (C) preferably has at least one hydrophobic group such as an alkyl group having 8 or more carbon atoms or an alkylsilicon group.

The present invention, in another aspect, provides spherical composite particles manufactured by the method according to the present invention. The present invention further provides, in still another aspect, a cosmetic including the spherical composite particles according to the present invention. The present invention also provides, in yet another aspect, a powder-layered manufacturing method using the spherical composite particles according to the present invention; and a molded article obtained by the method.

### Advantageous Effects of Invention

The method according to the present invention allows stable and easy manufacture of an organic solid powder (spherical composite particles) which structurally internally includes or externally bears a filler, or which internally includes a filler and externally bears another filler. This allows simple impartment of desired functions to the surface and/or inside of spherical composite particles and thereby efficiently gives spherical composite particles to be used in a wide variety of applications. The spherical composite particles help to give cosmetics having a variety of functions, contribute to process improvements in a powder-layered manufacturing method, and help to impart functions to a molded article obtained by the powder-layered manufacturing method.

### Brief Description of Drawings

[Fig. 1] Fig. 1 is a transmission electron micrograph of spherical composite particles obtained in Example 2.
[Fig. 2] Fig. 2 is a transmission electron micrograph of spherical composite particles obtained in Example 3.
[Fig. 3] Fig. 3 is a transmission electron micrograph of spherical composite particles obtained in Example 4.

### Description of Embodiments

The meltable organic solid (A) for use in the present invention is selected from thermoplastic resins, unvulcanized rubbers and thermoplastic elastomers.

Examples of the thermoplastic resins include, but are not limited to, polyamide resins, polyester resins, polyurethane resins, styrenic resins, acrylic resin, olefinic resins, vinyl resins, cellulose resins, polyether resins, various elastomers, polyarylates, poly(phenylene sulfide)s, polysulfones, poly(ether sulfone)s, polycarbonates, poly(phenylene ether)s, and poly(ether ether ketone)s.

The polyamide resins can be any polyamide resins such as polyamides prepared through polymerization of a diamine component (e.g., hexamethylenediamine or another alkylenediamine having 4 to 10 carbon atoms) with a dicarboxylic acid component (e.g., adipic acid or another alkylenedicarboxylic acid having 4 to 20 carbon atoms); polyamides prepared through polycondensation of an
aminocarboxylic acid (e.g., ω-aminoundecanoic acid or another aminocarboxylic acid having 4 to 20 carbon atoms); polyamides prepared through ring-opening polymerization of a lactam (e.g., ω-laurolactam or another lactam having 4 to 20 carbon atoms); and polyesteramides prepared through polycondensation of a diamine component (e.g., hexamethylenediamine or another alkylenediamine having 4 to 10 carbon atoms), a dicarboxylic acid component (e.g., adipic acid or another alkylenedicarboxylic acid having 4 to 20 carbon atoms), and a diol component (e.g., ethylene glycol or another alkylenediol having 2 to 12 carbon atoms). Polyamide resins include homopolyamides and copolyamides. Typical polyamide resins include aliphatic polyamides such as polyamide 46, polyamide 6, polyamide 66, polyamide 612, polyamide 610, polyamide 910, polyamide 912, polyamide 1212, polyamide 1012, polyamide 1010, polyamide 11, polyamide 12, polyamide 6T, and polyamide 9; aromatic polyamides such as aramid resin; and copolyamide resins.

The polyester resins can be any polyester resins such as polyesters prepared through polycondensation of a diol component and a dicarboxylic acid component; polyesters prepared through polycondensation of a hydroxycarboxylic acid; polyesters prepared through ring-opening polymerization of a lactone; and polyesters containing urethane bonds and prepared through a reaction between a polyesterdiol and a diisocyanate. Polyester resins include homopolyesters and copolyesters. Typical polyester resins include aliphatic polyesters such as polycaprolactones and poly(lactic acid)s; and aromatic polyesters such as poly(ethylene terephthalate)s, poly(butylene terephthalate)s, and polycaprolactones.

Exemplary polyurethane resins include resins prepared through a reaction of a diisocyanate, a polyol, and, according to necessity, a chain extender (e.g., ethylene glycol or ethylenediamine). Typical polyurethane resins include urethane resins prepared by using a soft segment composed typically of a polyester diol, polyether diol, or polycarbonate diol with a hard segment composed typically of an isocyanate (e.g., diphenylmethane diisocyanate (MDI), tolylene diisocyanate (TDI), isophorone diisocyanate (IPDI), hydrogenated MDI, or hexamethylene diisocyanate (HDI)),'a low-molecular-weight diamine, or a glycol.

Exemplary styrenic resins include styrenic polymers such as polystyrenes and α-methylstyrene polymers; copolymers of styrene or a derivative thereof with, for example, (meth)acrylic acid, (meth)acrylic acid ester, or acrylonitrile; and high-impact polystyrene (HIPS) resins and acrylonitrile-butadiene-styrene (ABS) resins derived from these styrenic resins, except for introducing a rubber component thereinto.

Exemplary acrylic resins include poly((meth)acrylic acid)s; poly(alkyl (meth)acrylate)s such as poly(methyl (meth)acrylate)s and poly(ethyl (meth)acrylate)s; polyacrylonitriles; and copolymers corresponding to these resins.

Exemplary olefinic resins include polyethylenes, polypropylenes, polybutenes, ethylene-propylene copolymers, and ethylene-acrylic acid copolymers.

Exemplary vinyl resins include homo- or co-polymers of vinyl compounds, such as vinyl acetate resins, vinyl chloride resins, vinylidene chloride resins, ethylene-vinyl acetate resins, poly(vinyl alcohol)s, fluorocarbon resins (e.g., tetrafluoroethylene resin), and vinyl ester resins.

Exemplary cellulose resins include cellulose homo- or co-polymers, such as cellulose acetate, cellulose acetate butyrate, and hydroxyethylcellulose.

Exemplary polyether resins include polyethylene glycols, polyethylene oxides, and polyoxymethylenes. Exemplary cellulose resins include cellulose esters such as cellulose acetate, cellulose acetate butyrate, and hydroxyethylcellulose; cellulose ethers; and cellulose carbamates. Exemplary polycarbonate resins include aromatic polycarbonates composed typically of bisphenol-A; and aliphatic polycarbonates composed of 1,6-hexamethylene glycol.

Further, unvulcanized rubbers and thermoplastic elastomers may be included. Exemplary rubbers include isoprene rubbers (IRs), butadiene rubbers (BRs), chloroprene rubbers (CRs), styrene-butadiene rubbers (SBRs), nitrile rubbers (NBRs), isobutylene-isoprene rubbers (IIRs), ethylene-propylene rubbers [e.g., ethylene-propylene copolymers (EPMs) and ethylene-propylene diene terpolymer rubbers (EPDMs)], acrylic rubbers (e.g., copolymers of acrylic acid ester and 2-chloroethyl vinyl ether (ACMs) and copolymers of acrylic acid ester and acrylonitrile (ANMs)), epichlorohydrin rubbers, silicone rubbers, fluorocarbon rubbers (FKMs), urethane rubbers (e.g., polyester-urethanes (AUs)), and chlorosulfonated polyethylenes (CSMs). Such rubbers are present as unvulcanized rubbers.

Thermoplastic elastomers can be any of well-known elastomers intermolecularly having a hard segment and a soft segment. Examples thereof include thermoplastic polyamide elastomers (TPAE), thermoplastic polyester elastomers (TPEE), thermoplastic polyurethane elastomers (TPU), thermoplastic polystyrene elastomers (TPS), thermoplastic fluorocarbon polymer elastomers, and polyolefin elastomers (thermoplastic olefin elastomers; TPO). Exemplary thermoplastic polyamide elastomers include polyamide elastomers having a polyamide component (e.g., polyamide 6 or polyamide 12) and a polyether component (e.g., polyether diol) as a hard segment and a soft segment, respectively.

The fillers (C) for use herein are not limited in their material and shape, as long as having a size smaller than that of the spherical composite particles to be manufactured. As used herein the term "filler" refers to a substance which is relatively inactive and which can impart various functions to the spherical composite particles. Examples of the functions include insulation properties, strength, viscosity, flame retardancy, electroconductivity, brightness, colorings (coloring properties), and antimicrobial properties. Fillers include inorganic or organic fillers. Exemplary inorganic fillers include calcium carbonate, magnesium carbonate, clay, kaolin, calcium phosphate, hydroxyapatite, mica, talc, silica, quartz powder, glass powder, diatomaceous earth, nepheline syenite, cristobalite, wollastonite, aluminum hydroxide, iron oxide, zinc oxide, titanium oxide, aluminum oxide, calcium sulfate, magnesium oxide, barium sulfate, dolomite, silicon carbide, silicon nitride, boron nitride, metal powders, graphite, ferrite, and carbon black (e.g., electroconductivity). Examples of fillers further include antimicrobial fillers (e.g., hydroxyapatite/silver and zeolite/silver) which are composed of a compound combined with a metal ion having an antimicrobial function, such as silver, copper, or zinc ion. Exemplary organic fillers include carbon black, carbon nanotubes, carbon fibers, organic pigments (e.g., phthalocyanine), and particles or granules of polymers such as crosslinked poly(methyl methacrylate)s.

Inorganic or organic colorants are preferably used as the fillers herein, because color development can be controlled as the colorants are immobilized on the surface or inside of particles composed of a thermoplastic resin. Exemplary inorganic colorants include inorganic pigments such as carbon black, titanium oxide, ultramarine blue, iron oxide red, black iron oxide, yellow oxide, chromium oxide, and multicomponent oxide pigments. Exemplary organic colorants include organic pigments including azo pigments; and polycyclic pigments such as anthraquinone, copper phthalocyanine blue, copper phthalocyanine green, quinacridone, and diketopyrrolopyrrole. Exemplary organic colorants further include disperse dyes and oil dyes for use in resins. In addition, tar pigments prescribed in Ordinance No. 30 of The Ministry of Health and Welfare of Japan (now The Ministry of Health, Labor, and Welfare of Japan) for use typically in cosmetics; and lake pigments such as aluminum lakes are also preferably used.

The shape of fillers for use herein may be any shape such as a spheroidal (e.g., spherical or oval spherical), circular cylindrical, or prismatic shape, as long as being granular or particulate. The size or dimensions of fillers for use herein may be chosen depending on the intended use, within a range not adversely affecting the dispersivity of the fillers. For example, the fillers may have a diameter or major axis of typically 0.001 to 100 µm, preferably 0.005 to 50 µm, and more preferably 0.010 to 30 µm.

The amount of fillers herein is typically about 1 to 80 parts by weight, and preferably about 5 to 60 parts by weight, per 100 parts by weight of the organic solid (A).

The spherical composite particles according to the present invention may further include additives in addition to the organic solid (A) and the filler(s) (C). Preferred examples of such additives include antioxidants, lubricants, weathering stabilizers, flame retardants, and other additives generally used in resins. The types and amounts of the additives may be chosen according to the required performance of the spherical composite particles.

For allowing the fillers to be internally included or externally born, the compatibility or miscibility between the filler(s) (C) and the organic solid (A) is very important. The interaction between the filler(s) (C) and the organic solid (A) is effectively indicated by a solubility parameter SP. When a difference in SP between the filler(s) (C) and the organic solid (A) is small, the filler(s) tends to be internally included in the particles, and when a difference in SP between them is large, the filler(s) tends to be externally born on the surface of the particles. It can be said that a difference in SP between them of less than 5 is a small difference in SP; and that a difference in SP between them of 5 or more is a large difference in SP. The SP may be determined according to a regular technique, such as the technique described in "SP Values, Basis, Applications, and Calculating Methods," (Hideki Yamamoto, Johokiko Co., Ltd.).

The difference in SP between the filler(s) (C) and the organic solid (A) may be controlled very effectively by treating inorganic fillers and organic fillers to be used as the fillers (C) typically with a surface treatment agent. For example, when treated with a treatment agent having a similar functional group to the functional group of the organic solid (A), or with a treatment agent having a functional group significantly interacting with the functional group of the organic solid (A), the filler (C) significantly interacts with the organic solid (A) and shows a small difference in SP from the organic solid (A). In contrast, when treated with a treatment agent having a functional group weakly interacting with the functional group of the organic solid (A), the filler (C) shows a large difference in SP from the organic solid (A). Typically, when the organic solid (A) has a carboxy group, including-type spherical composite particles may be obtained by treating the filler (C) with an aminosilane coupling agent so as to give a small difference in SP between the two components due to strong interaction between them. In contrast, outer-bearing-type spherical composite particles may be obtained by subjecting the filler (C) to siliconization typically with a polydimethylsiloxane so as to give a large difference in SP between the two components due to weak interaction between them. In addition, the combination use of a first filler treated with an aminosilane coupling agent and a second filler siliconized gives spherical composite particles which internally include the first filler and externally bear the second filler.

The method according to the present invention is carried out by melting and kneading the meltable or fusible organic solid (A), the component (B) immiscible with the organic solid (A), and the one or more fillers (C) having specific differences in SP from the organic solid (A) to form an organic solid composition which is composed of the organic solid (A) and the filler(s) (C) and is dispersed in a matrix composed of the component (B); and removing the matrix component typically with water and/or a solvent.

The component (B) for use in the present invention is not limited, as long as being immiscible or incompatible with the organic solid (A), and can be any well-known substance. The component (B) may be one which allows the formation of a dispersed structure of the organic solid composition according to the compositional ratio among the organic solid, the component (B), and the filler(s) (C) when these components are melted and kneaded. A component having a suitable melt viscosity may be chosen as the component (B) to give spherical composite particles having a desired particle size.

The component (B) can be either a water-soluble material or water-insoluble material. Exemplary water-soluble materials include saccharides such as monosaccharides, oligosaccharides, polysaccharides, sugar alcohols, polydextroses, maltodextrin, and inulin; hydrogenated products and hydrolyzed products of the saccharides; polyethylene oxides; polyethylene glycols; poly(vinyl alcohol)s; and cellulose derivatives. Exemplary water-insoluble materials include solid alcohols such as pentaerythritol, dipentaerythritol, stearic acid, adipic acid, dodecanedioic acid, and calcium stearate; carboxylic acids; metallic soaps; solid polyethylene wax, petroleum resins, polystyrenic resins, polyester resins, polycarbonate resins, acrylic resins, phenol resins, poly(vinyl butyral) resins, urethane resins, phenoxy resins, and other thermoplastic resins soluble in organic solvents. In the case of whichever of a water-soluble material or water-insoluble material, the material preferably shows a viscosity as low as possible when dissolved typically in water or a solvent, for easier removal of the component (B) in a subsequent purification step.

The immiscibility between the organic solid (A) and the component (B) may be verified by the following phenomenon (i) or (ii). Specifically, (i) when the organic solid (A), the component (B), and the filler(s) (C) are melted and kneaded with one another to give an organic solid composition, the organic solid composition has a melting point or glass transition point approximate (e.g., ±20%) to the melting point or glass transition point of the organic solid (A) alone or of the component (B) alone; or (ii) when the component (B) and about 20 percent by weight of the organic solid (A) relative to the component (B) are heated, kneaded with each other, and cooled to give a kneaded article, and the kneaded article is dissolved in a solvent which does not dissolve the organic solid (A) but dissolves the component (B) therein to give a dispersion, the dispersion is a dispersion in which the organic solid (A) is dispersed, when observed under a microscope. The glass transition point may be measured according to customary techniques such as differential scanning calorimetry (DSC) and dynamic viscoelastic measurement.

The organic solid composition herein includes a matrix composed of the component (B), and spherical composite particles which are composed of the organic solid (A) and the filler(s) (C) and which are dispersed in the matrix. The organic solid composition may be prepared according to well-known processes, such as a process of previously forming spherical composite particles in granular or particulate form, and dispersing the spherical composite particles in the component (B); and a process of melting and kneading the organic solid (A), the component (B), and the filler(s) (C) with one another and extruding the kneaded article. An organic solid composition prepared according to the latter process is preferably used in the present invention.

A preferred organic solid composition may be prepared, for example, by melting and kneading the organic solid (A), the component (B), the filler(s) (C), and, according to necessity, additives with one another to form a dispersion of composite particles which are spheroidal (e.g., spherical) and have substantially equal sizes, and extruding the dispersion. The melting, kneading, and extrusion may be performed typically using a regular kneader or extruder (e.g., single-screw extruder, twin-screw extruder, or roller extruder).

The kneading and extrusion temperature is not limited, as long as being a temperature at which the organic solid (A) and the component (B) are melted and being a temperature equal to or lower than the upper temperature limits (heat-proof temperatures) of the respective components. The temperature may be suitably set according typically to the types and amounts of the organic solid (A) and the component (B) and is typically about 100°C to 300°C and preferably about 110°C to 250°C. The viscosity of the kneaded article upon extrusion is preferably from 1 to 10000 Pa·s in terms of melt viscosity measured at the extrusion temperature with a capillograph. The melt viscosity is important for the control of the particle size of the target spherical composite particles. The extruded organic solid composition may have any shape such as a strand or sheet form. The extruded composition is preferably cooled immediately after extrusion.

The process gives an organic solid composition including a matrix composed of the component (B), and spherical composite particles dispersed in the matrix, which spherical composite particles are composed of the organic solid (A) and the filler(s) (C). Where necessary, the organic solid composition may be subjected to a customary molding/processing process such as melting, stretching, or compression.

The method according to the present invention is a method for manufacturing spherical composite particles by removing the component (B) from the organic solid composition having the above structure. The removal of the component (B) may be performed, for example, by a washing process of the organic solid composition, namely, by a process in which the organic solid composition is dispersed in a solvent to dissolve the component (B) in a liquid phase, and the spherical composite particles in a solid phase are recovered or collected. The solvent is not limited, as long as capable of dissolving not the organic solid (A) but the component (B). Examples of the solvent include water; water-soluble organic solvents such as lower alcohols; and mixtures of them. Among them, water is preferably employed from the viewpoints of environmental friendliness and good handleability.

The dispersing may be performed using customary mixing/agitating devices such as agitators and ultrasonic generators. Among them, an ultrasonic generator is preferably employed for higher dispersion efficiency and shorter process time.

The spherical composite particles recovered through the process (step) are dried to give product spherical composite particles. The ultimately obtained spherical composite particles may have any size which is not limited, can be chosen according to the intended use of the spherical composite particles, and is typically about 0.01 to 100 µm, and preferably about 0.5 to 80 µm. The size of the spherical composite particles can be controlled, for example, by adjusting conditions and parameters such as the types and amounts (compounding ratios) of the organic solid (A) and the component (B), the structure of the extruder, and the extrusion conditions.

The method according to the present invention gives spherical composite particles composed of the organic solid (A), and the filler(s) (C) suitably externally born on the surface of the organic solid (A) and/or suitably internally included in the organic solid (A). The presence of the filler(s) (C) on the surface or inside of the spherical composite particles may be verified or detected typically by observation under a scanning electron microscope (SEM) or transmission electron microscope (TEM).

The spherical composite particles according to the present invention have the above configuration and can thereby have desired functions as efficiently imparted on the surface or inside thereof. Examples of the functions include insulation properties, strength, viscosity, flame retardancy, electroconductivity, brightness, colorings (coloring properties), and antimicrobial properties. The spherical composite particles are usable typically as cosmetics (e.g., scrubs and foundation creams), fillers, agents for use in resinous membranes such as sheets and films and for imparting optical properties thereto, and various additives.

The cosmetics according to the present invention contain spherical composite particles manufactured by the method. The cosmetics according to the present invention may further contain, according to the intended use, customary additives such as alcohols, methylcellulose, CMC (carboxymethylcellulose), and other stabilizers, surfactants, flavors, preservatives, antioxidants, collagens, ultraviolet absorbers, mucopolysaccharides, binders, and extenders. These cosmetics are used typically in the form of liquids such as lotions, emulsions, creams, and gels, or in the form of solids such as powders, granules, pastes, and molded articles. They are usable typically as creams, foundation creams, lip creams, hand creams, body creams, toilet soap, cleaning agents, exfoliating cleansers, scrub cleansers, cataplasms (beauty packs), toning lotions (face lotions), milky lotions, beauty essences, shampoos, hair rinses or hair conditioners, and hair dressing products. Above all, the spherical composite particles according to the present invention are advantageously usable as foundation creams excellent in color development by using a colorant as the filler.

The powder-layered manufacturing method according to the present invention uses spherical composite particles obtained by the manufacturing method. The powder-layered manufacturing method according to the present invention contributes to process improvements by choosing the type of the filler externally born on the spherical composite particles. For example, the flowability of the powder in each layer may be improved and/or the adhesiveness of the powder (particles) with each other may be controlled. In addition, the method is very effective as a method for manufacturing a wide variety of three-dimensional articles being rich in functionality with efficient control, because the method allows the control of the strength, thermal stability, and mechanical properties of the resulting molded article by selecting the type of the filler internally included in the particles according to the intended use.

The molded article obtained by the powder-layered manufacturing method includes the spherical composite particles obtained by the manufacturing method. The molded article according to the present invention may further contain any of customary additives such as flame retardants, antioxidants, stabilizers, ultraviolet absorbers, and lubricants, according to the intended use. The molded article may further contain any other fillers such as glass, metals, metal oxides, and ceramics, in addition to the fillers (C). The spherical composite particles according to the present invention, when using colorants as fillers, are particularly advantageously usable in the manufacture of molded articles which require satisfactory coloring properties or dye sensitizing properties.

### EXAMPLES

The present invention will be illustrated in further detail with reference to several working examples below. It should be noted, however, that these examples are never construed to limit the scope of the present invention.

### EXAMPLE 1 (Reference Example)

In a twin-screw extruder set at 200°C, 20 parts by weight of a polyamide 12 resin (containing COOH terminal groups in a content of 123 mmol/kg and having a SP of 21.7) supplied by Daicel-Evonik Ltd., an oligosaccharide as a mixture of 65 parts by weight of a powdered hydrogenated glucose syrup (trade name "PO-10") supplied by Towa Chemical
Industry Co., Ltd. and 35 parts by weight of D-sorbitol, and 4 parts by weight of zinc oxide treated with stearic acid (having a particle size of 0.3 µm and a SP of 18.7) were melted, kneaded with one another, then extruded into strands (about 3 mm in diameter), cooled on a belt cooler, and thereby yielded pellets of an organic solid composition composed of the polyamide 12 resin, oligosaccharides, and zinc oxide.

Next, the pelletized organic solid composition was dissolved in water in a concentration of 5 percent by weight, subjected to filtration under reduced pressure using a 5A filter paper on a Nutsche, and washed. This process was repeated a total of 10 times to remove the water-soluble oligosaccharides from spherical composite particles composed of the polyamide 12 resin and the zinc oxide to thereby give a wet cake. The wet cake was dried at 80°C under reduced pressure

The resulting spherical composite particles had a median diameter of 5 µm. The spherical composite particles were observed under a transmission electron microscope (TEM) to find that they were spherical composite particles composed of polyamide 12 resin particles and, internally included therein, zinc oxide.

### EXAMPLE 2 (Reference Example)

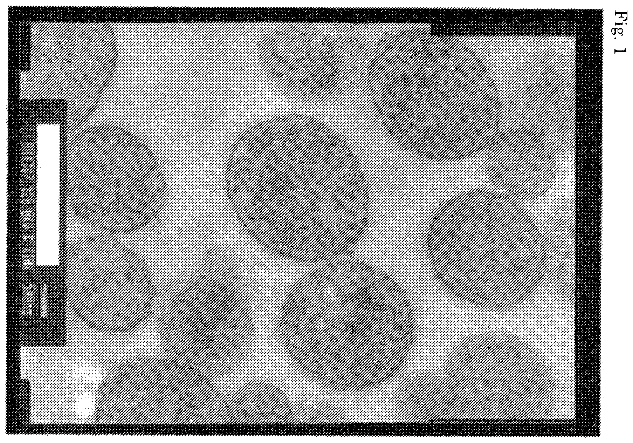

An organic solid composition was prepared, from which spherical composite particles were recovered by the procedure of Example 1, except for using 4 parts by weight of titanium oxide treated with calcium stearate (having a particle size of 0.3 µm and a SP of 18.0) instead of the zinc oxide and using a twin-screw extruder set at 210°C. The spherical composite particles had a median diameter of 1.6 µm. The spherical composite particles were observed under a transmission electron microscope (TEM) to find that they were spherical composite particles composed of polyamide 12 resin particles and, internally included therein, titanium oxide. The transmission electron micrograph of the obtained spherical composite particles is shown as Fig. 1.

### EXAMPLE 3 (Reference Example)

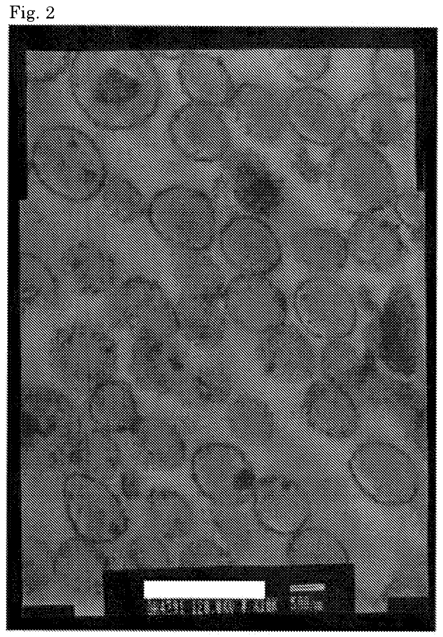

An organic solid composition was prepared, from which spherical composite particles were recovered by the procedure of Example 1, except for using 4 parts by weight of a siliconized titanium oxide (having a particle size of 0.3 µm and a SP of less than 10) instead of the zinc oxide and using a twin-screw extruder set at 190°C. The resulting spherical composite particles had a median diameter of 1.3 µm. The spherical composite particles were observed under a transmission electron microscope (TEM) to find that they were spherical composite particles composed of polyamide 12 resin particles and, immobilized on the surface thereof, titanium oxide. The transmission electron micrograph of the obtained spherical composite particles is shown as Fig. 2.

### EXAMPLE 4

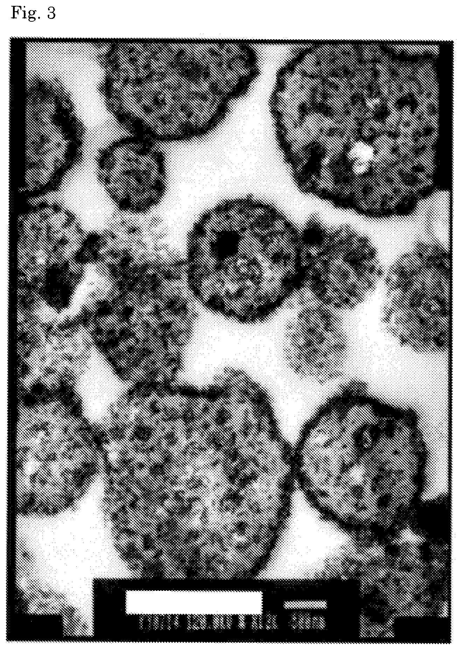

An organic solid composition was prepared, from which spherical composite particles were recovered by the procedure of Example 1, except for using, instead of the zinc oxide, 2 parts by weight of a siliconized titanium oxide (having a particle size of 0.3 µm and a SP of less than 10) and 2 parts by weight of calcium-stearate-treated titanium oxide (having a particle size of 0.3 µm and a SP of 18.0). The resulting spherical composite particles had a median diameter of 2.3 µm. The spherical composite particles were observed under a transmission electron microscope (TEM) to find that they were spherical composite particles composed of polyamide 12 resin particles; the titanium oxide immobilized on the surface of the resin particles; and the titanium oxide internally included in the resin particles. The transmission electron micrograph of the obtained spherical composite particles is shown in Fig. 3.

## Claims

1. A method for manufacturing spherical composite particles, the method comprising:
melting and kneading a meltable organic solid (A), a component (B) immiscible with the organic solid (A), and one or more fillers (C) having specific differences in solubility parameter SP from the organic solid (A) to give spherical composite particles comprising the organic solid (A) and the filler(s) (C) and being dispersed in the component (B),
wherein the meltable organic solid (A) is selected from thermoplastic resins, unvulcanized rubbers and thermoplastic elastomers,
wherein a first filler having a difference in SP of less than 5 and a second filler having a difference in SP of 5 or more are used as the fillers (C) to give the spherical composite particles which internally include the first filler (C) and externally bear the second filler (C),
wherein the filler(s) (C) comprises a filler whose surface has been treated with a substance having a specific difference in SP from the organic solid (A), and
wherein the solubility parameter SP is determined according to the technique described in "SP values, basis, applications, and calculating methods; Hideki Yamamoto, Johokiko Co. Ltd., issued on March 31, 2005".

2. The method according to claim 1, wherein the filler(s) (C) comprises, at least at its surface, a filler having a specific difference in SP from the organic solid (A).

3. The method according to claim 1, wherein the organic solid (A) comprises a thermoplastic resin.

4. The method according to claim 3, wherein the thermoplastic resin comprises a polyamide resin.

5. The method according to claim 1, wherein the filler(s) (C) has at least one selected from the group consisting of carboxyl groups, carboxylic acid groups, carboxylic acid salts, amino groups, amide groups, epoxy groups, and other hydrophilic groups.

6. The method according to claim 1, wherein the filler(s) (C) has at least one selected from the group consisting of alkyl groups having 8 or more carbon atoms, alkylsilicon groups, and other hydrophobic groups.

7. Spherical composite particles manufactured by the method as claimed in any one of claims 1 to 6.

8. A cosmetic comprising the spherical composite particles as claimed in claim 7.

9. A powder-layered manufacturing method, comprising carrying out powder-layered manufacturing using the spherical composite particles as claimed in claim 7.

10. A molded article manufactured by the powder-layered manufacturing method as claimed in claim 9.

## Patentansprüche

1. Verfahren zum Herstellen von sphärischen Kompositpartikeln, wobei das Verfahren umfasst:
Schmelzen und Kneten eines schmelzbaren organischen Feststoffs (A), einer Komponente (B), die mit dem organischen Feststoff (A) nicht mischbar ist, und eines oder mehreren Füllstoffen (C), besitzend spezifische Unterschiede im Löslichkeitsparameter SP vom organischen Feststoff (A), um sphärische Kompositpartikel zu erhalten, umfassend den organischen Feststoff (A) und den Füllstoff/die Füllstoffe (C) und dispergiert in Komponente (B),
wobei der schmelzbare organische Feststoff (A) ausgewählt ist aus thermoplastischen Harzen, unvulkanisierten Gummis und thermoplastischen Elastomeren,
wobei ein erster Füllstoff, besitzend einen Unterschied im SP von weniger als 5, und ein zweiter Füllstoff, besitzend einen Unterschied im SP von 5 oder mehr, als die Füllstoffe (C) verwendet werden, um die sphärischen Kompositpartikel zu erhalten, die intern den ersten Füllstoff (C) beinhalten, und extern den zweiten Füllstoff (C) aufweisen,
wobei der Füllstoff/die Füllstoffe (C) einen Füllstoff umfasst/umfassen, dessen/deren Oberfläche mit einer Substanz behandelt wurde, besitzend einen spezifischen Unterschied im SP vom organischen Feststoff (A), und
wobei der Löslichkeitsparameter SP gemäß der Technik bestimmt wird, die in "SP values, basis, applications, and calculating methods; Hideki Yamamoto, Johokiko Co. Ltd., veröffentlicht am 31. März 2005" beschrieben ist.

2. Das Verfahren gemäß Anspruch 1, wobei der Füllstoff/die Füllstoffe (C), zumindest an dessen/deren Oberfläche, einen Füllstoff, besitzend einen spezifischen Unterschied im SP vom organischen Feststoff (A) umfasst/umfassen.

3. Das Verfahren gemäß Anspruch 1, wobei der organische Feststoff (A) ein thermoplastisches Harz umfasst.

4. Das Verfahren gemäß Anspruch 3, wobei das thermoplastische Harz ein Polyamidharz umfasst.

5. Das Verfahren gemäß Anspruch 1, wobei der Füllstoff/die Füllstoffe (C) mindestens eines, ausgewählt aus der Gruppe, bestehend aus Carboxylgruppen, Kohlensäuregruppen, Salzen von Kohlensäure, Aminogruppen, Amidgruppen, Epoxygruppen und anderen hydrophilen Gruppen besitzt/besitzen.

6. Das Verfahren gemäß Anspruch 1, wobei der Füllstoff/die Füllstoffe (C) mindestens eines, ausgewählt aus der Gruppe, bestehend aus Alkylgruppen, besitzend 8 oder mehr Kohlenstoffatome, Alkylsilikongruppen und anderen hydrophoben Gruppen besitzt/besitzen.

7. Sphärische Kompositpartikel, hergestellt durch das Verfahren, wie in irgendeinem der Ansprüche 1 bis 6 beansprucht.

8. Kosmetikum, umfassend die sphärischen Kompositpartikel, wie in Anspruch 7 beansprucht.

9. Pulverbeschichtungsherstellungsverfahren, umfassend das Durchführen einer Pulverbeschichtung unter Verwenden der sphärischen Kompositpartikel, wie in Anspruch 7 beansprucht.

10. Geformter Gegenstand, hergestellt durch das Pulverbeschichtungsherstellungsverfahren, wie in Anspruch 9 beansprucht.

## Revendications

1. Procédé de fabrication de particules composites sphériques, le procédé comprenant :
la fusion et le malaxage d'un solide organique fusible (A), d'un constituant (B) non miscible au solide organique (A) et d'une ou plusieurs charges (C) ayant des différences spécifiques de paramètre de solubilité SP par rapport au solide organique (A) pour obtenir des particules composites sphériques comprenant le solide organique (A) et la ou les charges (C) et se trouvant dispersées dans le constituant (B),
où le solide organique fusible (A) est choisi parmi des résines thermoplastiques, des caoutchoucs non vulcanisés et des élastomères thermoplastiques,
où une première charge ayant une différence de SP inférieure à 5 et une seconde charge ayant une différence de SP de 5 ou plus sont utilisées en tant que charges (C) pour obtenir les particules composites sphériques qui incluent de façon interne la première charge (C) et portent de façon externe la seconde charge (C),
où la ou les charges (C) comprennent une charge dont la surface a été traitée avec une substance ayant une différence spécifique de SP par rapport au solide organique (A), et
où le paramètre de solubilité SP est déterminé selon la technique décrite dans « SP values, basis, applications, and calculating methods [Valeurs, base, application et méthodes de calcul du SP] ; Hideki Yamamoto, Johokiko Co. Ltd., publié le 31 mars 2005 ».

2. Procédé selon la revendication 1, dans lequel la ou les charges (C) comprennent, au moins au niveau de leur surface, une charge ayant une différence spécifique de SP par rapport au solide organique (A).

3. Procédé selon la revendication 1, dans lequel le solide organique (A) comprend une résine thermoplastique.

4. Procédé selon la revendication 3, dans lequel la résine thermoplastique comprend une résine polyamide.

5. Procédé selon la revendication 1, dans lequel la ou les charges (C) comportent au moins un élément choisi dans le groupe constitué de groupes carboxyle, groupes acide carboxylique, sels d'acide carboxylique, groupes amino, groupes amide, groupes époxy et d'autres groupes hydrophiles.

6. Procédé selon la revendication 1, dans lequel la ou les charges (C) comportent au moins un élément choisi dans le groupe constitué de groupes alkyle ayant 8 atomes de carbone ou plus, groupes alkylsilicium et d'autres groupes hydrophobes.

7. Particules composites sphériques fabriquées par le procédé comme revendiqué dans l'une quelconque des revendications 1 à 6.

8. Cosmétique comprenant les particules composites sphériques comme revendiqué dans la revendication 7.

9. Procédé de fabrication par stratification de poudre, comprenant la mise en oeuvre d'une fabrication par stratification de poudre utilisant les particules composites sphériques comme revendiqué dans la revendication 7.

10. Article moulé fabriqué par le procédé de fabrication par stratification de poudre ainsi que revendiqué dans la revendication 9.
